# EUROPEAN PATENT SPECIFICATION

(11) **EP 4 401 743 B1**
(45) Date of publication and mention of the grant of the patent: **03.09.2025**
(21) Application number: 22789675.0
(22) Date of filing: 15.09.2022
(51) Int. Cl.: A61K 31/198, A61K 31/728, A61K 9/00, A61P 19/00, A61P 19/02, A61P 19/08, A61K 47/18, A61K 47/36

(54) **COMPOSITION COMPRISING SODIUM HYALURONATE AND AMINO ACIDS FOR USE IN THE ORTHOPEDIC FIELD**
ZUSAMMENSETZUNG MIT NATRIUMHYALURONAT UND AMINOSÄUREN ZUR VERWENDUNG IM ORTHOPÄDISCHEN BEREICH
COMPOSITION COMPRENANT DU HYALURONATE DE SODIUM ET DES AMINOACIDES POUR UNE UTILISATION DANS LE DOMAINE ORTHOPÉDIQUE

(30) Priority: 16.09.2021 IT 202100023894
(43) Date of publication of application: 24.07.2024
(73) Proprietor: Professional Derma SA, 6900 Lugano (CH)
(72) Inventor: CONTI, Edoardo, 6900 Lugano-Paradiso (CH)
(74) Representative: Pinnarò, Chiara
(86) International application number: PCT/IB2022/058720
(87) International publication number: WO 2023/042120

(56) References cited:
- WO-A1-2007/073921
- WO-A1-2014/172784

## Description

### Technical field

The present invention relates to injectable compositions comprising specific combinations of low and high molecular weight hyaluronic acid (HA), non cross-linked, in combination with a specific composition of amino acids (AA) for use in the medical field, in particular in the treatment of osteoarthrosis and damage to the cartilage caused by trauma.

### State of the art

Osteoarthrosis is the most common joint disease among the rheumatic disorders affecting the Western world.

It is a chronic degenerative joint disease that can be widespread or localized, affecting the cartilage of the diarthrodial or synovial joints; in these joints the normal metabolic process of the chondrocytes is impaired, leading to softening, fibrillation, ulceration and subsequent sclerosis of the subchondral bone, and in the final stages to the formation of new bone and subchondral cysts.

The clinical picture is initially dominated by pain, typically mechanical, which subsides with rest. After a long inactivity, for instance in the morning or after sitting for a long time or post-inactivity, affected individuals may experience painful spasms which are however short-lived and subside with walking. Pain can be triggered by the use of stairs, especially downhill, squatting, especially in the case of patellofemoral osteoarthrosis, or with prolonged use of vehicle pedals. Initially there may also be pain in the periarticular areas and a modest effusion. The pain may later affect the entire joint, become nocturnal and be accompanied by frequent joint effusions. Functional impairment appears only in a late stage.

The ideal treatment of osteoarthritis, particularly of the knee, requires a combination of pharmacological and other treatments, such as rehabilitation, use of aids (walking sticks, orthotics or knee braces) and weight loss, designed as based on patient needs based on local risk factors (obesity, mechanical factors, physical activity), general risk factors (age, comorbidities, multiple therapy), pain intensity levels and degree of disability, signs of inflammation (effusion), location and degree of structural damage.

The main therapy is analgesic, with the administration of painkillers as needed to limit pain and allow joint movement. Paracetamol is the drug of choice for pain control; in the case of medium and severe pain, it is possible to alternate or combine the use of paracetamol with that of NSAIDs which are considered for patients who do not respond to paracetamol and for patients at gastrointestinal risk. Sometimes, the pain that accompanies arthrosis is so severe that opioid drugs are needed. The use of corticosteroids is limited to rare cases of inflammation associated with arthrosis.

In case of localized arthrosis at the level of a specific joint (e.g. hips, knees, hands) it is possible to use hyaluronic acid (HA), which, together with chondroprotective drugs, is usually used in the infiltrative treatment: these active materials, in addition to delaying the damage to the cartilages, are able to protect the joints, making them less fragile. HA, a fundamental constituent of normal joints that appears quantitatively reduced and qualitatively altered in osteoarthrosis and in some of the conditions predisposing it, represents an important therapeutic aid especially in the treatment of some categories of patients, having proved to be safe and effective in clinical practice. Similarly, this treatment can be performed to counteract the effects of cartilage damage caused by trauma. In fact, hyaluronic acid has a direct action on chondrocytes and synovitics and consequently beneficial effects on the structure of the cartilage.

During the osteoarthrosis process, both the mechanical and biological properties of HA (pain relieving, anti-inflammatory and immunosuppressive) are reduced; there is numerous experimental evidence to support this thesis (Nicholls MA, Fierlinger A, Niazi F, et al. The disease-modifying effects of hyaluronan in the osteoarthritic disease state. Clin Med Insights Arthritis Musculoskelet Disord 2017; 10: 1-10) (Itman RD, Manjoo A, Fierlinger A, et al. The mechanism of action for hyaluronic acid treatment in the osteoarthritic knee: a systematic review. BMC Musculoskelet Disord 2015; 16: 321).

With regard to its pain-relieving properties, results of studies on animals with experimental arthritis have indicated that the intra-articular injection of HA reduces the activity of the primary afferents related to pain both basically and during movement. The results of a study indicated that, unlike what happens with anti-inflammatory drugs, the reduction of pain resulting from the administration of HA is associated with preservation of cartilage and carries out anti-inflammatory actions. Oxidative stress plays an important role in osteoarthrosis and can be induced by an abnormal cyclic load on the joint. Reactive oxygen species (ROS) reduce the synthesis of proteoglycans and accelerate the senescence of chondrocytes and therefore their tissue repairing capacity. Results of various studies have shown that HA reduces ROS levels and protects chondrocytes from the negative consequences of exposure to these molecules. Finally, there is evidence that indicates that HA has a protective role at the tissue level. During the progression of osteoarthrosis or following a trauma, the extracellular matrix (ECM) of the cartilage is remodeled by proteases expressed by chondrocytes in response to inflammation. The modifications of the ECM alter the biomechanical environment of the chondrocytes and cause a progression of the disease. Results of various studies indicate that exogenous HA can increase the synthesis of ECM molecules. The exogenous HA also stimulates the synovial fibroblasts to produce new HA. Finally, the administration of HA is able to increase the synthesis of proteoglycans in cartilage subjected to mechanical stress.

It is known that amino acids, the building blocks of proteins, when placed as substrates, stimulate the translation of proteins. However, to obtain the desired effect it is necessary to investigate specific amino acid compositions which, only if correctly identified and administered, can favor the synthesis of a particular subset of proteins. To date, there are some compositions used to counteract oxidative stress and tissue damage; these compositions are in particular made to be administered by injection.

Specifically, a composition comprising sodium hyaluronate of a single molecular weight and a mixture of amino acids which includes Glycine, L-Proline, L-Leucine, L-Lysine hydrochloride.

The viscosupplementing effect of this composition is quite limited and of short duration and this means that the treatment must be repeated frequently enough to give effective functional benefit or effective relief to the patient There is therefore a need for more effective compositions to treat osteoarthrosis and damage to cartilage caused by trauma.

WO 2014/172784 A1 discloses a cross-linked hyaluronic acid composition comprising from 0.5 to 2.0% w/w of hyaluronic acid and from 0.1 to 5.0% w/w of at least one biologically active compound, which can be an amino acid.

WO 2007/073921 A1 refers to compositions comprising hyaluronic acid and clodronic acid in form of injection solutions for intra-articular administration for use in the treatment of osteoarthritis.

### Summary of the invention

It is an object of the present invention an injectable composition comprising a specific combination of low and high molecular weight hyaluronic acid (HA), non cross-linked, in combination with a specific composition of amino acids (AA).

In particular, the composition according to the present invention comprises:
- Non cross-linked sodium hyaluronate with a 100-400 kDa molecular weight, in a concentration between 7 and 20 mg/ml
- Non cross-linked sodium hyaluronate with a 2000 kDa or higher molecular weight, in a concentration between 10 and 25 mg/ml
- a mixture of amino acids comprising:
   Glycine 6-12.5 mg/ml
   L-Proline and/or L-Hydroxyproline 5-8 mg/ml
   L-Alanine 1-5 mg/ml
   L-Valine 1-5 mg/ml
   L-Leucine 1-5 mg/ml
   L-Lysine HCl (hydrochloride) 1-5 mg/ml
   L-Arginine HCl (hydrochloride) 1-5 mg/ml

Compared to the known art compositions, the composition according to the invention has alanine, valine and arginine hydrochloride, this mixture has an improved effect and is particularly effective in reducing painful symptoms in patients.

In addition, the viscosupplementing effect achieved thanks to the two different molecular weights in high concentration allows the product to have a longer duration and the patient to have a longer lasting result.

In a preferred embodiment, the composition according to the invention comprises:
9 mg/ml of Glycine
6.5 mg/ml of Proline
2 mg/ml of Alanine
2.5 mg/ml of Valine
1 mg/ml of Leucine
2.5 mg/ml of L-Lysine HCl (hydrochloride)
1.5 mg/ml of L-Arginine HCl (hydrochloride)

In one embodiment the composition according to the invention comprises sodium hyaluronate with a 100-400 kDa molecular weight, present in a concentration of 16 mg/ml and sodium hyaluronate with a 2000 kDa molecular weight, present in a concentration of 16 mg/ml.

In one embodiment, sodium hyaluronate with a 100-400 kDa molecular weight is present in a concentration of 12 mg/ml and sodium hyaluronate with a 2000 kDa molecular weight is present in a concentration of 20 mg/ml.

In a preferred embodiment, the composition according to the invention comprises a total sodium hyaluronate concentration per ml greater than 25 mg/ml, preferably greater than or equal to 32 mg/ml.

In a preferred embodiment, the composition has a pH preferably between 6.8 and 7.5, even more preferably between 7 and 7.3.

In a preferred embodiment, the composition according to the invention comprises at least one of pharmaceutically acceptable excipients or adjuvants, a buffer, preferably phosphate buffer, an anesthetic agent, preferably a local anesthetic agent.

It is an object of the present invention an injectable composition as described above for use in the medical field.

In particular, it is an object of the present invention an injectable composition as described above for use in orthopedics, preferably for the treatment of osteoarthrosis or damage to the cartilage caused by trauma.

The present invention also relates to a kit comprising an injectable composition as described above, comprised in a pre-filled syringe and optionally comprising the instructions for use.

It is also an object of the present invention the use of the compositions as described above for use in the medical field, in particular in the treatment of deterioration and/or senescence of the articular cartilage.

Further objects will become apparent from the detailed description which follows.

### Description of the figures

Figure 1 - Distribution of the injection site for the patients included in the study.
Figure 2 - Results Graph VAS Pain Scale, ROM Impairment, ADL and Sport Impairment before and after treatment.
Figure 3 - Painkillers before treatment, at 10 days and 30 days.
Figure 4 - Side effects of the treatment.

### Detailed description of the invention:

A new composition suitable for intra-articular injection was therefore developed by the inventor comprising a specific combination of medium and high molecular weight, non cross-linked hyaluronic acid (HA), in combination with a specific composition of amino acids (AA), effective in the treatment of osteoarthrosis, and in general of joint problems due to damage to the cartilage caused by trauma (injuries during sports or accidents in general). This composition comprises:
- hyaluronic acid having a 100-400 kDa molecular weight
- non cross-linked hyaluronic acid having a 2000 kDa molecular weight or higher
- 25 mg/ml total of amino acids thus present
   Glycine 6-12.5 mg/ml
   L-Proline and/or L-Hydroxyproline 5-8 mg/ml
   L-Alanine 1-5 mg/ml
   L-Valine 1-5 mg/ml
   L-Leucine 1-5 mg/ml
   L-Lysine HCl (hydrochloride) 1-5 mg/ml
   L-arginine HCl (hydrochloride) 1-5 mg/ml

In a preferred embodiment the composition comprises:
9 mg/ml of Glycine
6.5 mg/ml of Proline
2 mg/ml of L-Alanine
2.5 mg/ml of L-Valine
1 mg/ml of L-Leucine
2.5 mg/ml of L-Lysine HCl
1.5 mg/ml of L-Arginine HCl

In a preferred embodiment, the composition is that shown in table 1.

**TABLE 1**

| **Composition** | mg/ml (MW) |
|---|---|
| Sodium hyaluronate | 7-20(100-400) |
| | 10-25 (≥ 2000) |
| Glycine | 9 |
| Proline | 6.5 |
| Alanine | 2 |
| Valine | 2.5 |
| Leucine | 1 |
| Lysine hydrochloride | 2.5 |
| Arginine hydrochloride | 1.5 |
| Total AA | 25 |

In a preferred embodiment, the composition according to the invention comprises a total sodium hyaluronate concentration per ml greater than 25 mg/ml, preferably greater than or equal to 32 mg/ml.

The composition according to the present invention may further comprise physiological solution, pharmaceutically acceptable excipients or adjuvants.

The composition according to the present invention can further comprise a buffer, for instance a phosphate buffer, to adjust the pH.

The pH of the composition is preferably between 6.8 and 7.5, even more preferably between 7 and 7.3.

The composition according to the present invention can further comprise an anesthetic agent, in particular a local anesthetic, preferably lidocaine, in a concentration comprised between 0.1% and 0.4%, preferably between 0.2% and 0.3%.

In order to verify the effectiveness of the composition, the inventors designed a study, enrolling 74 patients with semi-acute and chronic joint pain due to joint trauma, symptomatic osteoarthritis or trauma-activated osteoarthrosis; patients were treated with 3 intra-articular injections of the composition according to the invention over a period of 30 days.

Patients' perceived pain (VAS scale), range of motion (ROM) and improvement in carrying out activities of daily living and in the ability to play sports were monitored. After 30 days there was a strong improvement in each of these parameters with in particular a very strong efficacy in the reduction of symptomatic pain (The timely reduction of pain was 30.7%, while at 30 days 82.7 %). The intake of painkilling drugs during the treatment period and the presence of possible side effects were also monitored. Before the start of treatment, 92% of patients habitually resorted to painkillers, after 30 days from the start of treatment only 5.3% of patients reported that they still felt the need to take them.

The inventor has therefore developed an injectable composition which, compared to the compositions currently described in the known art, has a superior efficacy in reducing painful symptoms, movement difficulties and therefore the use of painkilling drugs in treated patients.

According to the present invention, "injectable" is intended to be delivered from syringes under normal conditions at normal pressure and refers to intra-articular injection at the desired destination site.

The composition according to the invention can be formulated for intra-articular administration both in the form of a viscous solution and in the form of reconstitutable powders, and is prepared according to conventional methods well known to the skilled in the art also comprising the addition of pharmaceutically acceptable excipients.

It is therefore an object of the present invention an injectable composition comprising:
- from 7 to 20 mg/ml of non cross-linked sodium hyaluronate with a 100-400 kDa molecular weight
- from 10 to 25 mg/ml of non cross-linked sodium hyaluronate with a 2000 kDa or higher molecular weight
- A mixture of amino acids comprising:
   Glycine 6-12.5 mg/ml
   L-Proline and/or L-Hydroxyproline 5-8 mg/ml
   L-Alanine 1-5 mg/ml
   L-Valine 1-5 mg/ml
   L-Leucine 1-5 mg/ml
   L-Lysine HCl (hydrochloride) 1-5 mg/ml
   L-arginine HCl (hydrochloride) 1-5 mg/ml

In a preferred embodiment the composition comprises:
9 mg/ml of Glycine
6.5 mg/ml of L-Proline
2 mg/ml of L-Alanine
2.5 mg/ml of L-Valine
1 mg/ml of L-Leucine
2.5 mg/ml of L-Lysine HCl
1.5 mg/ml of L-Arginine HCl

In a preferred embodiment the composition comprises 16 mg/ml of non cross-linked sodium hyaluronate with a 100-400 kDa molecular weight and 16 mg/ml of non cross-linked sodium hyaluronate with a 2000 kDa or higher molecular weight.

In another preferred embodiment, the composition according to the invention comprises 12 mg/ml of non cross-linked sodium hyaluronate with a 100-400 kDa molecular weight and 20 mg/ml of non cross-linked sodium hyaluronate with a 2000 kDa molecular weight.

In a preferred embodiment, the composition comprises a total sodium hyaluronate concentration per ml greater than 25 mg/ml, preferably greater than or equal to 32 mg/ml.

The composition according to the present invention can further comprise at least one of physiological solution, pharmaceutically acceptable excipients or adjuvants.

The composition according to the present invention can further comprise a buffer, for instance a phosphate buffer, to adjust the pH. Said pH is preferably between 6.8 and 7.5, even more preferably between 7 and 7.3.

The composition according to the present invention can further comprise an anesthetic agent, in particular a local anesthetic, preferably lidocaine, in a concentration comprised between 0.1% and 0.4%, preferably between 0.2% and 0.3%.

Therefore, it is an object of the present invention a composition as described above for use in the medical field; the use of said composition for use in the treatment of osteoarthrosis and other joint problems due to damage to the cartilage caused by trauma.

It is an object of the present invention the composition for use in particular in the treatment of osteoarthrosis of the knee, ankle, foot, hip and shoulder.

The present invention also relates to a kit comprising an injectable composition according to the invention in pre-filled interarticular injection syringe and optionally the instructions for use.

### EXAMPLES

### Introduction and study design:

74 consecutive patients were treated with a combination of 2.5 ml of hyaluronic acid and amino acids and a local anesthetic.
32 mg/ml of sodium hyaluronate with low (16 mg/ml) and high (16 mg/ml)
molecular weight
9 mg/ml of Glycine
6.5 mg/ml of Proline
2 mg/ml of Alanine
2.5 mg/ml of Valine
1 mg/ml of Leucine
2.5 mg/ml of Lysine HCl
1.5 mg/ml of Arginine HCl

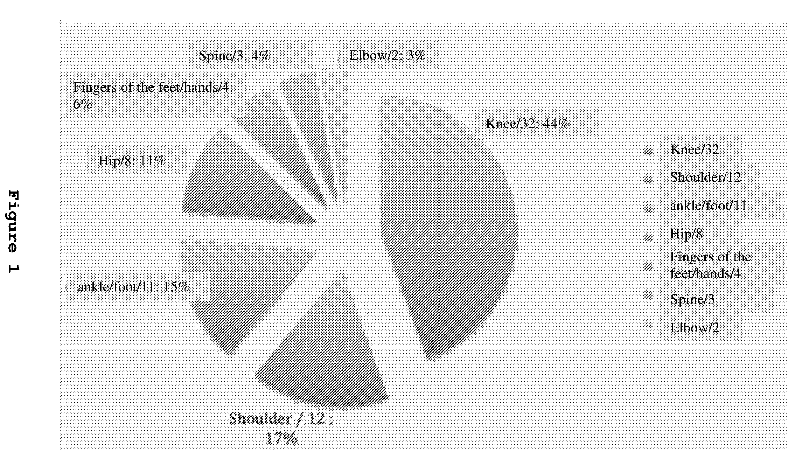

Consecutive, non-randomized patients were included who experienced semi-acute and chronic joint pain for a minimum of 2 weeks due to joint trauma, symptomatic osteoarthrosis, or trauma-activated osteoarthrosis and who would otherwise have been treated with oral medications, intra-joints injections, physiotherapy or surgery. The exclusion criteria were recent traumatic joint injuries and open wounds, bacterial infections and patients with grade IV arthrosis. The mean age of the patients was 59.6 years, the oldest patient was 80 and the youngest 19. 35 patients were male, 39 female. All major joints were treated, involving 32 knees, 12 shoulders, 11 ankles, 8 hips and 4 toes/hands, 3 spinal joints and 2 elbows (as shown in Figure 1). The mean degree of arthritis was 1.9. The reported pain duration was 12.7 months, the shortest two weeks (acute pain) and the longest over 48 months (chronic pain).

The treatment was carried out in a private orthopedic practice, where the patients were physically examined and questioned by the physician; patients signed a written consent form before each injection.

A questionnaire was prepared for the patient to be filled out by the physician during three subsequent treatments, the parameters recorded before treatment were the duration of pain, intensity of pain, impaired range of motion of the affected joint, impaired activities of daily living, the impairment of sports activities in the past 4 weeks and the current intake of oral pain medication.

About 10 days after the first injection, patients were asked to quantify pain intensity, pain medication intake and early side effects.

Approximately 30 days after the last injection, patients were again asked to describe pain intensity, current range of motion of the joint, impaired activities of daily living, impaired sports activities, side effects/symptoms, tolerance of treatment, satisfaction with treatment and whether they would recommend it to friends or family. The purpose of this follow-up was to determine the possible pain relief, safety, side effects and tolerance of this new treatment combination.

### Method:

All patients received a series of 3 injections of the product described above over an approximate time frame of 30 days; each injection was scheduled approximately 7-10 days apart. The joint was disinfected, cooled with ice spray and initially a small amount of local anesthetic was injected into the subcutaneous space in order to eliminate the small particles of skin, then the hypodermic needle was inserted into the joint space, where the residue local anesthetic was injected. With the hypodermic needle firmly in place, the first syringe was removed and subsequently the composition was injected through the needle positioned as the second component. The hypodermic needle was removed and the joint was treated with a small dressing. The treated joint was then moved back and forth by the physician in order to distribute the injected components throughout the joint space. Patients were advised not to take hot baths, saunas or direct sunbathing for 7 days following treatment.

### Results:

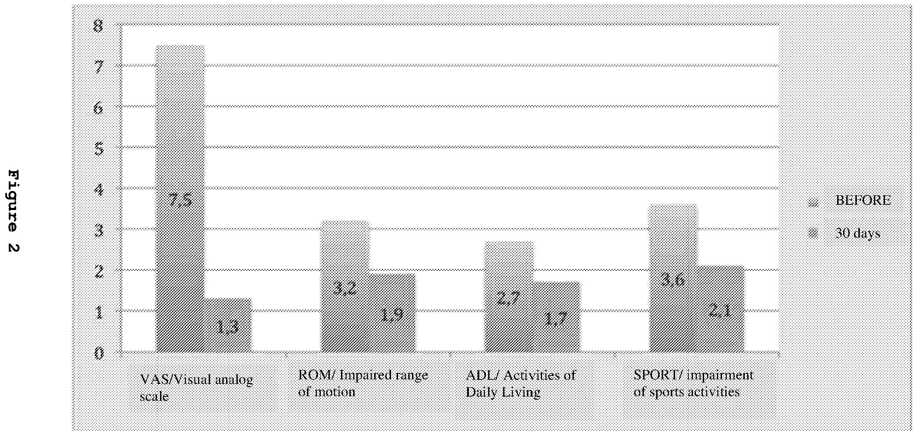

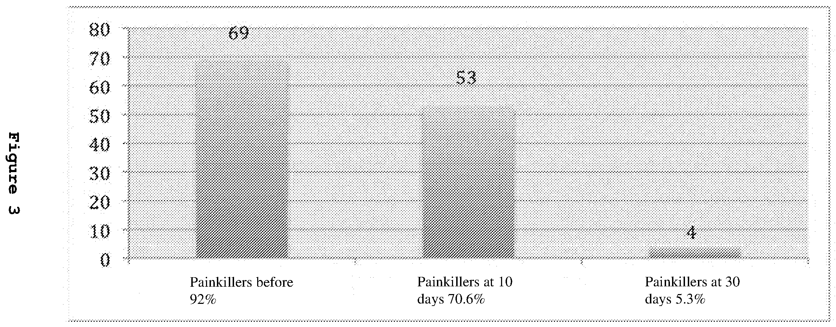

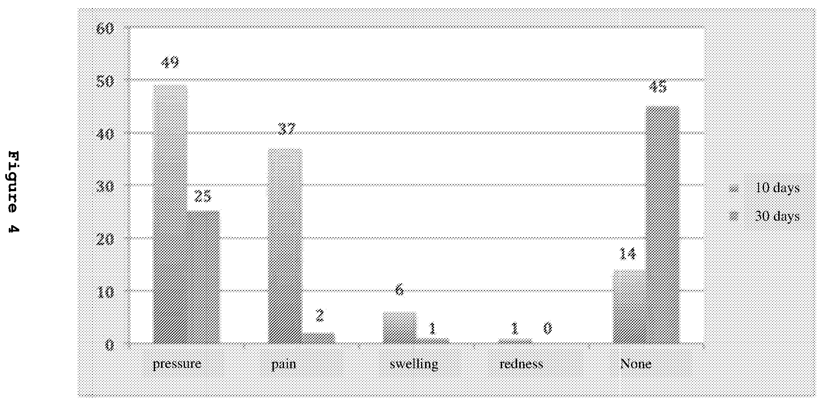

**1. Duration of pain:** The mean duration of pain was 12.7 months.
**2. Pain intensity/VAS:** The mean pain reported before treatment, as recorded with the VAS pain scale (VAS = visual analog scale; pain level 0 = no pain, 10 = the strongest pain imaginable) was 7.5, a rather severe pain. At the same question 10 days after the first injection, the mean pain had dropped to 5.2 on the VAS scale, a mean pain, at about 30 days after therapy (about 30 days after the last injection) the mean pain had dropped to 1.3 on the VAS scale, a very mild pain. The timely reduction of pain was 30.7%, while at 30 days it was 82.7%.
**3. Impaired range of motion (ROM):** Patients were asked about their impaired range of motion (ROM) of the affected joint with four possible responses: unaffected (1), mildly impaired (2), moderately impaired ( 3) and severely impaired (4). The mean mobility impairment was 3.2 before treatment and 1.9 30 days after treatment, an improvement from moderate to mild impairment of 40.6% (Figure 2).
**4. Activities of Daily Living, ADL:** The same was asked about the impaired activities of daily living (ADL), the mean ADL impairment was 2.7 before treatment and 1.7 after 30 days, with a 37% reduction (Figure 2).
**5. Sports Activities:** Patients were asked about current sports activities, which were severely impaired 3.6 at the time of interview, prior to treatment, and improved to 2.1 at 30 days, an improvement of 41.6 % (figure 2).
   Figure 2 shows the results graph VAS pain scale, ROM, ADL and Sport impairment before and after treatment.
**6. Painkillers:** 69 (92%) of the 74 patients had taken painkillers prior to treatment, 5 did not. Medication intake after 10 days was prolonged by 53 (70.6%) patients, 21 of whom reported no longer relying on painkillers. 30 days after treatment 4 patients (5.3% of the group) needed oral painkillers, 71 patients did not need painkillers at the time of the interview (Figure 3).
**7. Side Effects:** The main side effects reported at the first interview, approximately 7-10 days after the first treatment, 49 patients reported pressure, 37 reported pain, 6 reported swelling, 1 reported redness at the injection site, 14 patients reported no noticeable side effects of the injection. Responding to 30-day remaining side effects, 25 patients reported residual pressure in the joint, 2 patients reported pain, 1 patient reported swelling, and 45 patients had no noticeable side effects or symptoms at the time of the interview. No infections or worsening of symptoms were noted during this investigation (Figure 4).
**8. Treatment tolerance:** Patients were asked how they tolerated the series of injections with possible response Excellent 1, Good 2, Fair 3, Poor 4. 67 patients responded that they tolerated excellently (1), seven patients tolerated it well (2).
**9. Subjective satisfaction:** When asked about their satisfaction with the outcome of the treatment series, 60 patients responded excellent (1) and 13 responded good (2) and one patient responded fair (3).
**10. Recommendation:** All 74 patients stated that they would recommend treatment to a friend or family member, or would reconsider having similar treatment in the future.

### Conclusion:

The series of minimally invasive intra-articular injections with the composition described above comprising a combination of hyaluronic acid and amino acids is a safe and well tolerated treatment. The series of treatments with the combination of hyaluronic acid and amino acids was very well tolerated by the patients and brought them very satisfactory results with a satisfactory reduction in pain of 82.7% as well as a significant reduction in pain medication in all cases treated and improvement in range of motion of affected joints by 68%. The side effects of the treatment were mild and well tolerated by the patients. No further worsening or worsening of symptoms was observed at the time of the interview, and no infections, allergic reactions or permanent damage were observed.

## Claims

1. An injectable composition comprising:
- non cross-linked sodium hyaluronate with a molecular weight between 100 and 400 kDa in a concentration between 7 and 20 mg/ml,
- non cross-linked sodium hyaluronate with a molecular weight at least 2000 kDa, in a concentration between 10 and 25 mg/ml,
- a mixture of amino acids consisting of Glycine in a concentration between 6 and 12.5 mg/ml, L-Proline and/or L-Hydroxyproline in a concentration between 5 and 8 mg/ml, L-Alanine in a concentration between 1 and 5 mg/ml, L-Valine in a concentration between 1 and 5 mg/ml, L-Leucine in a concentration between 1 and 5 mg/ml, L-Lysine hydrochloride in a concentration between 1 and 5 mg/ml, L-arginine hydrochloride in a concentration between 1 and 5 mg/ml.

2. The composition according to the preceding claim wherein the amino acid mixture consists of 9 mg/ml of Glycine, 6.5 mg/ml of L-Proline, 2 mg/ml of L-Alanine, 2.5 mg/ml of L-Valine, 1 mg/ml of L-Leucine, 2.5 mg/ml of L-Lysine HCl, 1.5 mg/ml of L-Arginine HCl.

3. The composition according to any one of the preceding claims, wherein sodium hyaluronate with a molecular weight between 100 and 400 kDa is present in a concentration of 16 mg/ml and sodium hyaluronate with a molecular weight of at least 2000 kDa is present in a concentration of 16 mg/ml.

4. The composition according to one of claims 1 or 2 wherein sodium hyaluronate with a molecular weight between 100 and 400 kDa is present in a concentration of 12 mg/ml and sodium hyaluronate with a molecular weight of at least 2000 kDa is present in a concentration of 20 mg/ml.

5. The composition according to one of claims 1 or 2 wherein sodium hyaluronate is present in a total concentration greater than 25 mg/ml preferably greater than or equal to 32 mg/ml.

6. The composition according to any one of the preceding claims, wherein said composition has a pH preferably between 6.8 and 7.5, even more preferably between 7 and 7.3.

7. The composition according to any one of the preceding claims comprising at least one of: physiological solution, pharmaceutically acceptable excipients or adjuvants, a buffer, preferably phosphate buffer, an anesthetic agent, preferably a local anesthetic agent, preferably lidocaine, in a concentration ranging from 0.1% to 0.4% preferably between 0.2% and 0.3%.

8. An injectable composition according to the preceding claims for use in the medical field.

9. The injectable composition according to the preceding claims for use in the orthopedic field, preferably for the treatment of osteoarthrosis and/or in the treatment of articular cartilage senescence and/or damage to the cartilage caused by trauma.

10. A kit comprising an injectable composition according to any one of claims 1 to 7, comprised in a pre-filled syringe and optionally comprising instructions for use.

## Patentansprüche

1. Injizierbare Zusammensetzung, bestehend aus:
- unvernetztem Natriumhyaluronat mit einem Molekulargewicht zwischen 100 und 400 kDa in einer Konzentration zwischen 7 und 20 mg/ml,
- unvernetztem Natriumhyaluronat mit einem Molekulargewicht von mindestens 2000 kDa in einer Konzentration zwischen 10 und 25 mg/ml,
- einer Aminosäuremischung bestehend aus Glycin in einer Konzentration zwischen 6 und 12,5 mg/ml, L-Prolin und/oder L-Hydroxyprolin in einer Konzentration zwischen 5 und 8 mg/ml, L-Alanin in einer Konzentration zwischen 1 und 5 mg/ml, L-Valin in einer Konzentration zwischen 1 und 5 mg/ml, L-Leucin in einer Konzentration zwischen 1 und 5 mg/ml, L-Lysinhydrochlorid in einer Konzentration zwischen 1 und 5 mg/ml, L-Argininhydrochlorid in einer Konzentration zwischen 1 und 5 mg/ml.

2. Zusammensetzung gemäß dem vorhergehenden Anspruch, wobei die Aminosäuremischung aus 9 mg/ml Glycin, 6,5 mg/ml L-Prolin, 2 mg/ml L-Alanin, 2,5 mg/ml L-Valin, 1 mg/ml L-Leucin, 2,5 mg/ml HCl L-Lysin und 1,5 mg/ml HCl L-Arginin besteht.

3. Zusammensetzung gemäß einem der vorhergehenden Ansprüche, wobei Natriumhyaluronat mit einem Molekulargewicht zwischen 100 und 400 kDa in einer Konzentration von 16 mg/ml und Natriumhyaluronat mit einem Molekulargewicht von mindestens 2000 kDa in einer Konzentration von 16 mg/ml vorliegt.

4. Zusammensetzung nach einem der Ansprüche 1 oder 2, wobei Natriumhyaluronat mit einem Molekulargewicht zwischen 100 und 400 kDa in einer Konzentration von 12 mg/ml und Natriumhyaluronat mit einem Molekulargewicht von mindestens 2000 kDa in einer Konzentration von 20 mg/ml vorliegt.

5. Zusammensetzung nach einem der Ansprüche 1 oder 2, wobei Natriumhyaluronat in einer Gesamtkonzentration von mehr als 25 mg/ml, vorzugsweise mehr als oder gleich 32 mg/ml vorliegt.

6. Zusammensetzung nach einem der vorhergehenden Ansprüche, wobei die Zusammensetzung einen pH-Wert vorzugsweise zwischen 6,8 und 7,5, noch bevorzugter zwischen 7 und 7,3 aufweist.

7. Zusammensetzung nach einem der vorhergehenden Ansprüche, enthaltend mindestens eines der folgenden: physiologische Lösung, pharmazeutisch verträgliche Trägerstoffe oder Adjuvantien, einen Puffer, vorzugsweise Phosphatpuffer, ein Anästhetikum, vorzugsweise ein Lokalanästhetikum, vorzugsweise Lidocain, in einer Konzentration von 0,1% bis 0,4%, vorzugsweise 0,2% bis 0,3%.

8. Injizierbare Zusammensetzung nach den vorhergehenden Ansprüchen zur Verwendung im medizinischen Bereich.

9. Injizierbare Zusammensetzung nach den vorhergehenden Ansprüchen zur Verwendung im orthopädischen Bereich, vorzugsweise zur Behandlung von Osteoarthrose und/oder zur Behandlung von Gelenkknorpelalterung und/oder traumabedingten Knorpelschäden.

10. Kit, enthaltend eine injizierbare Zusammensetzung nach einem der Ansprüche 1 bis 7, in einer Fertigspritze und optional mit Gebrauchsanweisung.

## Revendications

1. Composition injectable comprenant:
- du hyaluronate de sodium non réticulé de poids moléculaire compris entre 100 et 400 kDa, à une concentration comprise entre 7 et 20 mg/ml,
- du hyaluronate de sodium non réticulé de poids moléculaire d'au moins 2000 kDa, à une concentration comprise entre 10 et 25 mg/ml,
- un mélange d'acides aminés composé de glycine à une concentration comprise entre 6 et 12,5 mg/ml, de L-proline et/ou de L-hydroxyproline à une concentration comprise entre 5 et 8 mg/ml, de L-alanine à une concentration comprise entre 1 et 5 mg/ml, de L-valine à une concentration comprise entre 1 et 5 mg/ml, de L-leucine à une concentration comprise entre 1 et 5 mg/ml, de chlorhydrate de L-lysine à une concentration comprise entre 1 et 5 mg/ml, de chlorhydrate de L-arginine à une concentration comprise entre 1 et 5 mg/ml.

2. Composition selon la revendication précédente, dans laquelle le mélange d'acides aminés est constitué de 9 mg/ml de glycine, 6,5 mg/ml de L-proline, 2 mg/ml de L-alanine, 2,5 mg/ml de L-valine, 1 mg/ml de L-leucine, 2,5 mg/ml de HCl de L-lysine et 1,5 mg/ml de HCl de L-arginine.

3. Composition selon l'une quelconque des revendications précédentes, dans laquelle l'hyaluronate de sodium de poids moléculaire compris entre 100 et 400 kDa est présent à une concentration de 16 mg/ml et l'hyaluronate de sodium de poids moléculaire d'au moins 2000 kDa est présent à une concentration de 16 mg/ml.

4. Composition selon l'une des revendications 1 ou 2, dans laquelle l'hyaluronate de sodium de poids moléculaire compris entre 100 et 400 kDa est présent à une concentration de 12 mg/ml et l'hyaluronate de sodium de poids moléculaire d'au moins 2000 kDa est présent à une concentration de 20 mg/ml.

5. Composition selon l'une des revendications 1 ou 2, dans laquelle l'hyaluronate de sodium est présent à une concentration totale supérieure à 25 mg/ml, de préférence supérieure ou égale à 32 mg/ml.

6. Composition selon l'une quelconque des revendications précédentes, dans laquelle ladite composition a un pH de préférence compris entre 6,8 et 7,5, plus préférentiellement entre 7 et 7,3.

7. Composition selon l'une quelconque des revendications précédentes, comprenant au moins un des éléments suivants: une solution physiologique, des excipients ou adjuvants pharmaceutiquement acceptables, un tampon, de préférence un tampon phosphate, un anesthésique, de préférence un anesthésique local, de préférence la lidocaïne, à une concentration comprise entre 0,1% et 0,4%, de préférence entre 0,2% et 0,3%.

8. Composition injectable selon les revendications précédentes, destinée à être utilisée dans le domaine médical.

9. Composition injectable selon les revendications précédentes, destinée à être utilisée dans le domaine orthopédique, de préférence pour le traitement de l'osthéoarthrose et/ou dans le traitement de la sénescence du cartilage articulaire et/ou des lésions cartilagineuses causées par un traumatisme.

10. Kit comprenant une composition injectable selon l'une quelconque des revendications 1 à 7, présentée dans une seringue préremplie et éventuellement accompagnée d'un mode d'emploi.
